# EUROPEAN PATENT APPLICATION

(11) **EP 1 098 198 A1**
(43) Date of publication of application: **09.05.2001**
(21) Application number: 00123709.8
(22) Date of filing: 31.10.2000
(51) Int. Cl.: G01N 33/53, G01N 33/68, G01N 33/577, C07K 16/42

(54) **Method for the qualitative and quantitative determination of class igG human antibodies**

(30) Priority: 05.11.1999 IT MI992319
(71) Applicant: DIESSE DIAGNOSTICA SENESE S.p.A., I-20123 Milano (IT)
(72) Inventor: Cocola, Francesco, 53018 Sovicille SI (IT); Berti, Brunilde, 53037 San Gimignano SI (IT); Soldatini, Claudia, 53035 Monteriggioni SI (IT); Paoli, Carlo, 50023 Impruneta FI (IT)
(74) Representative: Mancini, Vincenzo, Dr.

(57) **Abstract**

The invention refers to a method for the qualitative and quantitative determination, in an analytical solid phase, of human class IgG antibodies specific for a given antigen.

## Description

The invention refers to a method for the qualitative and quantitative determination of class IgG human antibodies.

The chemical conjugation of an enzyme to an antibody or to an antigen allows detection of the formation of immune complexes on a solid phase in as far as the enzyme, following conjugation, binding to the solid phase and removal by washing of the reagents in excess, leads to the formation by subsequent interaction with the substrate of a coloured product which is the object of qualitative and quantitative determinations by means of optical density readings. This approach presents many of the characteristics of an ideal immunological assay; it is versatile, simple, makes use of stable, economical reagents and, through the use of a solid phase, allows the simple separation (generally, by means of washing) of the components which have reacted, in other words which have been bound, from those left "free".

The analytical techniques have been optimized to reach a level of sensitivity comparable to those obtained with radioimmunological assays, using extremely small volumes of the sample to be analysed and making possible the performance of a large number of analyses at the same time.

This technical progress has had a considerable impact in epidemiology and in the diagnosis of infectious diseases, while these methods can also be used for the detection and measurement of pharmaceuticals, hormones, for screening of monoclonal antibodies, and for many other applications.

In particular, the use of monoclonal antibodies with defined specificity and homogeneous affinity, has lead to the introduction of many new asays, for example the detection of antigens of the acquired immunodeficiency syndrome virus(AIDS).

Antibody determination (IgG, IgM, IgA etc.) in the field of infectious diseases is generally performed using various kinds of experimental methods, known as "sandwich", "capture" or "competitive" tests. The competitive methods do not allow the assay of class-specific immunoglobulins. In the case of IgM, but also IgG, the technique which offers greatest specificity and sensitivity is the "capture" method, although the most widely used for IgG is the "sandwich" method.

The immunological techniques for the qualitative and quantitative determination of specific antibodies generally include the addition of the test sample to the antigen which is fixed on the solid phase (the excess antigen is removed previously by washing the solid phase); the amount of specific antibody which remains bound to the antigen, after washing to remove any in excess which has remained free, can be determined using labelled antibodies (tracer) which bind specifically to the antibody to be detected. In order to increase the sensitivity of the analytical technique, it is possible to use enzymes such as HRP or alkaline phosphatase as tracers.

Alternatively, it is possible to perform a true capture: specific antibodies for the Ig class of interest first adsorb the immunoglobulins to be assayed, so that the antigen which is subsequently added is captured only by the antibodies of interest, revealing the bound antigen through the aid of labelled antibodies able to recognize the antigen.

In detail, in the "sandwich" enzyme immunoassays for the determination of specific antibodies, a solid phase is used which is generally composed of a microtiter plate, on which antigens characteristic of the infecting antigen arecoated. The coating can also be performed using other suitable recipients and materials such as glass tubes, cellulose, polystyrene beads etc.

In the sandwich test procedure which is generally used for IgG, the antigen coated on the support is incubated with the test sample, which is generally serum or plasma and which may contain the specific antibodies to be detected, in order to facilitate binding between the two components constituting the reaction. After the first incubation period, the specific antibodies for a given antigen will be bound, if present, to the solid phase and can be washed to eliminate all the other antibodies which will not have been bound. This is considered the first analytical step. In the subsequent step, a tracer composed of a labelled antibody (generally labelled with an enzyme: peroxidase, etc.) able to recognize and bind the antibodies still on the support, is left to react for a period of time sufficient for this purpose. The second analytical step is concluded with the washing of the wells and subsequent elimination of the unbound tracer. In the third and final step an enzyme detector (substrate) enables the specific antibodies bound to the antigen to be measured.

There are numerous applications of this general principle, by means of which, in addition to the IgG antibodies, IgA or IgM directed against viral, bacterial or protozoal antigens can be assayed. In all these cases the detection of the antigen/antibody complex on the solid phase takes place after removal (through washings) of the antibodies which have not reacted.

It is also known that the structure of IgG-class antibodies changes when they are bound to their antigen [N. Marquart, J. Deisenhofer, "Immunol. Today", 3, 160 (1982); D.R. Davies, H. Metzger, "Ann. Rev. Immunol." 1, 87 (1983); J. C. Jaton "Immunohemotherapy. A guide to immunoglobulin prophylaxis and therapy". Academic Press, London 1981, p. 14].

Experimental methods, denominated "one step", are also known, which include the combination of the first two analytical steps; in the case of antibodies, these techniques are only applicable in "competitive" methods. In consideration of the fact that any immunoglobulin which binds to the antigen, independent of the class to which it belongs, is able to "compete" with the tracer, these techniques have to be considered inadequate for the determination of immunoglobulins of a specific class [D. Catty, "Antibodies: A practical approach" vol. II, 1989, ed. IRL Press, Oxford; T. Porstmann, S. T. Kiessig, "J. Immunological Methods" 150, 5 (1992); J. P. Gosling, "Clin. Chem." 36, 1408 (1990)].

The tracers used at the present time never allow the "target" to be selected, i.e. by binding only the IgG which have reacted with the antigen.

Surprisingly, an enzyme immunoassay method has now been found for the qualitative and quantitative determination of human IgG class antibodies in a test sample, comprising the use of a monoclonal antibody which reacts selectively with IgG, in which said monoclonal is directed towards a conformational epitope of the Fc fragment of human IgG which is exposed by binding with an antigen specific for the IgG and binds selectively to the IgG which have reacted with said antigen.

In a preferred embodiment, the method according to the invention includes the adhesion of an antigen specific for IgG to a solid phase, contact of the resulting solid phase with the test sample in a mixture with the monoclonal antibody to which a tracer has been bound, either directly or indirectly, incubation of the solid phase comprising the sample/monoclonal-tracer mixture, and washing of the resulting final mixture.

In another preferred embodiment, the method according to the invention includes coating of the monoclonal antibody on the solid phase; mixing of the antigen to which a tracer is bound either directly or indirectly, with the sample; incubation of the solid phase including the sample/antigen-tracer mixture; and washing of the resulting final mixture.

The invented method includes a single incubation phase and a single washing phase.

The tracer can be bound to the antibody or the antigen either directly or indirectly; in other words, both the antibody and the antigen can be, for example, respectively labelled or conjugated.

In another preferred embodiment, the tracer which can be used to advantage for realization of the invented method is, for example, an enzyme, selected from the group composed of peroxidase, alkaline phosphatase, urease, glucosidase; in particular, horse radish peroxidase (HRP).

In the case that the invented method is realized according to the "capture" method, this includes coating a solid phase, for example the walls of the microtiter wells, with the monoclonal antibody defined above; mixing of the antigen, to which a tracer is bound either directly or indirectly, with the sample, preferably in a volume ratio from 1:5 to 1:25, in particular, 1:10, and over a period ranging from 15 to 60 minutes, allowing the formation of the immune complex between the antigen and IgG in the sample, such immune complex thus being captured by the solid phase; incubation of the solid phase including the sample/antigen-tracer mixture; and washing of the resulting final mixture.

The single wash of the solid phase has the purpose of removing the excess tracer that is not bound to the solid phase.

According to the above illustration, a further embodiment of the invention consists in a monoclonal antibody which must be directed towards a conformational epitope of the Fc fragment of human IgG and binds selectively to the IgG which have reacted with the antigen that is specific for the human IgG; bearing in mind these characteristics, an expert in the field can select a suitable monoclonal antibody to realize the invented method. Preferably, the monoclonal antibody is an isotype 2a immunoglobulin, produced by means of a hybridoma known as "IgG 38-77" (deposited at the Centre of Advanced Biotechnologies (CBA), Interlab Cell Line Collection - Biotechnologies Service - Largo R. Benzi, 10 - 16132 Genova - Italy, on August 10 1999, number PD99002), which preferentially recognizes class IgG human antibodies which have reacted with the antigen practically without binding to any of the others. (A 25% increase in IgG in the reaction mixture causes a decrease in signal by about 15%). The antibodies with the characteristics illustrated allow the invented method to be accomplished in a single analytical step instead of two, requiring a single incubation step and wash step.

The preferred technique for the method according to the invention is the enzyme immunoassay technique, in particular according to the "Elisa" test (Enzyme-Linked Immunosorbent Assay) which makes use of microtiter plates for the solid phase.

The method according to the invention allows a single incubation step and a single wash step to be performed, because all the components necessary for performance of the assay can be mixed.

In fact, in a single incubation the specific antibodies in the test sample are bound to the solid phase and at the same time are revealed by the monoclonal antibody, to which a tracer is bound either directly or indirectly as defined previously, which binds only to the IgG which have reacted with the antigen. In this single incubation step, the same reactions that normally take place in the "classic" two-phase techniques, occur without interfering with each other.

The invented method therefore allows the "target" to be selected, i.e. only the IgG which have reacted with the antigen are in fact bound.

The specificity of the monoclonal antibody (and of the tracer) for the immune complex which is formed between the IgG in the sample and the antigen on the solid phase, allows an evident simplification of the procedure.

In the preferred case, in which the conjugation of the monoclonal antibody produced from Ig 38-77 is achieved with HRP, the monoclonal-tracer thus obtained is mixed with the test sample (human serum or plasma) in a volume ratio from 1:5 to 1:25, in particular 1:10, performing the incubation, preferably in 15-60 minutes, in a well coated with the antigen. During the incubation the specific IgG in the sample are bound to the antigen and are then recognized by the monoclonal antibody/tracer. Those that are free in the reaction medium are eliminated together with the excess tracer in the single wash step required in the method.

Another embodiment of the invention includes a reagent for the qualitative and quantitative enzyme immunoassay of class IgG human antibodies, which includes the monoclonal antibody defined above.

According to another embodiment, the invention also includes a kit for the qualitative and quantitative enzyme immunoassay of class IgG human antibodies, which includes the reagent of the invention; in particular, a tracer, for example an enzyme, is bound either directly or indirectly to the reagent. In order to accomplish the present invention the enzyme, which can be selected from those already indicated above, is preferably HRP.

Of the different immunological analytical methods known, the "sandwich" and "capture" enzyme immunoassays and agglutination methods, if classified according to the reaction pathway, or immunofluorescent, radioimmunological methods etc. if classified according to the tracer, all prove to be suitable for realization of the method according to the present invention.

The following examples illustrate the invention without limiting it.

### Example 1

### Production of the monoclonal antibody called IgG 38-77

### Preparation of a monoclonal antibody anti-human IgG bound to the antigen.

### 2. Preparation of the hyperimmune spleens

4 female Balb/c mice aged 6 weeks were immunized with human IgG (Sigma, cod. I 4506) at a dose of 50 µg in 200 µl of Complete Freund Adjuvant (CFA, produced by Sigma Chemical Co.) by intraperitoneal injection (i.p.). After 15 days the same treatment was repeated in all mice. The first boost was performed after 45 days by injecting intravenously 100 µg in 100 µl of PBS in each mouse; the second booster dose was given after 49 days, by intravenous injection of 50 µg in 200 µl of PBS in each mouse. The spleens were removed three days after the last treatment.

### 3. Fusion

In order to obtain the hybridoma (splenocyte x myeloma) it is necessary to remove the unwanted cells because cell fusion is a casual process which can take place between myeloma cells, between splenocytes and between splenocytes and myeloma: a selective culture medium containing hypoxanthine-thymidine-aminopterin (HAT) was utilized for this purpose.

Aminopterin inhibits dihydrofolate-reductase, which synthesizes purine and pyrimidinic bases from simple compounds. However, the cells can make use of alternative salvage pathways, based on the TK (thymidin-kinase) and HGPRT (hypoxanthin-guanidine phosphorybosil transferase) enzymes. This metabolic pathway is operative when the precursors hypoxanthin (H) and thymine (T) are available in the tissue culture, and allows the normal cells to overcome the metabolic inhibition. A deficit in one of the TK or HGPRT genes leads to the impossibility to make use of the alternative pathway. The myeloma used for fusions is generally deficient in one of these enzymes (generally HGPRT, in as far as the gene is situated on the X chromosome and therefore a singe mutation is sufficient to inactivate it). In the case of somatic fusion between splenocytes and myeloma, genomic complementation occurs by the splenic parental cells, which allows the metabolic blockage to be overcome. The myeloma cells which have not become fused die because of the presence of HAT, the splenocytes die because they are unable to grow *in vitro*.

The materials and methods used are described.

### 3.6. Culture media:

RPMI (Roswell Park Memorial Institute): this abbreviation identifies the medium used for cell growth and multiplication of tumoral lines
RPMI 1640 (Gibco, code 51800-035);
RPMI 1640, supplemented with NaHCO₃ (23 mM), Hepes buffer (Gibco, code 066-01344A) 10 mM, L-glutamine (Sigma, code G-5763) 1.5 mM, penicillin-streptomycin (Seromed, A-2213) 100 IU/ml, gentamycin (Sigma, code G-1272) 20 µg/ml.

After dissolving the powders, the pH was brought to 7.2 with 40% NaOH, filtered sterilely with a 0.2 µ filter (Costar, 8002) and stored at +4°C.

### RPMI + FCS (Fetal Calf Serum)

RPMI-Base, prepared as reported above was supplemented with 10% FCS v/v (Seramed, tested for hybridomas).

### RPMI + HAT (hypoxanthine-thymidine-aminopterin) (selective medium)

2 mL of a 0.05 mM solution of aminopterin (A), 2 mL of a 0.15 mM solution of thymidine (T) and 10 mM of hypoxanthine (H) were added to 200 mL of RPMI + FCS, prepared as reported above.

### RPMI + HT (hypoxanthine + Thymidine)

The medium was prepared as reported above, with the exception of aminopterin.

### PEG (polyethylene glycol) 1550 solution

3 mL of RPMI-FCS medium were added to 2 mL of PEG (1550 Serva) 40% weight/volume.

### 3.7. Plates with macrophages

These are supporting cells which facilitate cell growth. The supporting (feeder) cells act as conditioners for the growth medium allowing the growth of the hybridomas at low concentrations. This role is performed by various cell lines, the most widely used system being murine macrophages obtained by peritoneal washing.

5 mL of RPMI + FCS were injected into the peritoneal cavity of a mouse. After gentle massaging of the abdomen, the liquid was aspirated with a sterile syringe and added to 45 mL of RPMI-FCS. This suspension was dispensed, 1 mL/well, in 24-well plates. The plates were incubated for about 24 h to favour adhesion of the macrophages to the bottom of the wells.

2.3. Cell fusion (J. H. Goding, Antibody production by Hybridomas, J. Immunological Methods 39 (1980) 285-308).

The non-secreting, 8-azaguanine resistent SP-02/0 Ag 14 myeloma line was used (ATCC cod. CRL-1581). The splenocytes obtained from the spleens of immunized mice (reported in paragraph 1.) were fused with the myeloma in a 2:1 ratio. First each cell line was washed in RPMI-FCS and then the two lines were combined in 50 mL centrifuge tubes (Costar 3251), in the ratio reported above, and centrifuged at 1500 rpm x 10*'*. After complete removal of the supernatant, 0.4 mL of a PEG solution were added to the cell pellet over 2' at 40°C with constant stirring. After a pause of 1' with continual stirring, the cell suspension was brought to 50 mL with RPMI+FCS 10% and dispensed 1 mL/well in two 24-well plates containing macrophages (2.2). The plates were incubated at 37°C in an atmosphere with 5% CO₂ and 100% humidity. After about 4 hours, the RPMI+HAT selective medium was added, 1 mL/well. The plates were left to incubate again at 37°C, with 5% CO₂ and 100% humidity. Growth of hybridomas was checked by inverse microscopy. 10-12 days after fusion and sufficient cell growth, the tissue culture supernatant of the wells with hybridoma growth was tested for the determination of specific antibodies in the indirect ELISA method described below.

### 2.6. Screening for families

Each 24-well plate shows growth of several individual clones which together represent a family of hybridomas.

### Method:

Nunc Maxisorp microtiter plates were sensitized separately with 100 µl/well of a 10 µg/mL solution of human IgG, IgA, IgM (Sigma), leading to coating of the Ig (IgG or IgA or IgM) on the solid phase. The supernatants of each family were added separately to each solid phase and incubated at 37°C for 1 hour. After several washings, 100 µl of tracer composed of goat anti-mouse IgG antibodies conjugated with alkaline phosphatase (Sigma code 4656) were added to each well, and incubated at 37°C for 1 hour. Subsequently, 100 µl of a 1 mg/mL solution of p-nitrophenylphosphate substrate (Sigma) in diethanolamine buffer were added to each well. The reaction was stopped after 30' with NaOH 1M and read at 405 nm. The families which were positive only to the test for human IgG were cloned.

2.5. Cloning by limiting dilution (J.H. Goding, Antibody production by Hybridomas, J. Immunological Methods 39 (1980) 285-308.

The cells of each well (family) that was positive in the ELISA test were harvested, counted with a Burker chamber, and diluted in RPMI+HT medium in order to obtain 100 cells in 10 mL of the same medium. 10µl/well were dispensed in 96-well plates (Costar) with macrophages (2.2). The plates were incubated at 37°C with 5% CO₂ and 100% humidity. After about 10 days, the wells with a single clone were detected by inverse microscopy. After complete metabolization the supernatant of each well was again tested in ELISA for the human IgG alone as reported in 2.4.

The clones which proved positive were expanded in 24-well plates. After adequate growth, the tissue supernatant of each expansion in 24 wells was retested in ELISA (2.4) on human IgG, IgA and IgM. The clones which were reconfirmed as being specific and with optimal binding characteristics for human IgG alone, were recloned (subclones) and processed as reported in 2.3, 2.4 and 2.5.

### 2.6. Selection of the clones of interest

The subclones which were derived from the second donation were studied with a selective method for their specific recognition of human IgG involved in a bond with the antigen. This selection was performed by testing all the tissue supernatants of the subclones in the ELISA system reported below:
Fc specific goat IgG anti-mouse IgG (Sigma 42-80) were coated at a concentration of 20 µg/mL on Nunc Maxisorp plates, 100 µl/well, and left overnight at room temperature. After elimination of the liquid phase and washings, 100 µl of undiluted supernatant, and diluted 1:200 for each subclone, were dispensed in 4 separate wells. Incubation was then performed for 1 hour at 37°C, during which the Mab present in the tissue culture supernatant of each subclone was captured by the Fc fragment, by means of the IgG coated on the solid phase.

At this point the experiment was performed in two different ways which will be indicated as "one-step" and "step by step". In the "one step" method, after suitable dilution (1:500) a human serum sample confirmed as positive for syphilis and a confirmed negative human serum were placed in separate wells together with the tracer composed of the 17K immunodominant protein for syphilis, obtained by recombinant technique and conjugated with HRP. After 1 h incubation at 37°C, elimination of the liquid and washing, the HRP substrate was added. The reaction was stopped after 15' by the addition of H₂SO₄ 0,3 M.

In the "step by step" method the serum was added separately at the same final dilution as that used in the well in the "one-step" method. A 1-hr incubation at 37°C followed. After elimination of the liquid phase and washing, the same tracer as that used in the "one-step" method was added at the same final dilution. After incubation for 1 h at 37°C and washings, the substrate was added and left for 15', after which the reaction was stopped with H₂SO₄ 0.3 M.

The results with the IgG 38-77 clone are reported in the table below, in comparison with another anti-IgG clone called 18-13, used as a parallel test:

| | ONE-STEP | | | STEP-BY-STEP | |
|---|---|---|---|---|---|
| | | OD | | OD | |
| | Dil. | + | - | + | - |
| IgG 38-77 | 1:200 | 2070 | 32 | 248 | 45 |
| IgG 18-13 | 1:200 | 158 | 38 | 456 | 44 |
| Dil. = diluition OD = optical density | | | | | |

The different behaviour of the two clones is evident. The IgG 38-77 clone allows use of the "one-step" technique. The other clone, previously selected only for its ability to recognize human IgG, is unsuitable.

### 2. Isotyping and characterization of the selected Mabs

Immunoglobulins or Ig are globular proteins formed of 4 polypeptide chains formed by 2 equal pairs, known as lambda and kappa, with different molecular weights. The lambda (light) chains are the same in all the "classes" or types of Ig. The kappa (heavy) chains show different aminoacid sequences and determine the class of immunoglobulin, i.e. IgG, IgA, IgM, IgD, IgE. Within the same class, for example the IgG, there may be slight differences in the sequence which in the mouse lead to 4 subclasses or isotypes called IgG₁, IgG₂ₐ, IgG_{2b} e IgG₃.

In accordance with the ELISA method described above and in order to determine the isotype, the supernatant of each hybridoma was added to a microplate coated with human IgG and, after washings, rabbit antisera anti-mouse isotype and then the tracer, composed of goat IgG anti-rabbit IgG conjugated with alkaline phosphatase (BioRad, kit Mouse-Typer cod. 172-2055), were added. The reaction was detected with p-nitrophenylphosphate as reported above (2.4.). Moreover, the supernatant of each hybridoma was tested against the Fc, F(ab)2 and Fab fragments of human IgG prepared according to the method described by J. W. Goding in "Monoclonal antibodies: Principles and Practice"; chapter 4, page 98-128, 1983. For this purpose, the Fab, F(ab)₂ ed Fc fragments, obtained by methods described in the bibliographical references reported above, were coated separately on Nunc Maxisorp plates code 469949 at 10 µg/ml. The supernatant of each clone was reacted separately with each fragment and the reaction was revealed by the addition of an enzyme tracer composed of goat IgG anti-mouse IgG conjugated with alkaline phosphate (Sigma code 4656).

The results are reported in the table below. The monoclonal antibody produced by the clone 18-13 was included in the same test, as it was able to bind human IgG. The clone 38-77 isotype proved to be of class IgG₂ₐ.

| | Isotype | Immunoreactivity | | | |
|---|---|---|---|---|---|
| | | IgG | F(ab)2 | Fab | Fc |
| IgG 18-13 | IgG 1 | + | - | - | + |
| IgG 38-77 | IgG 2a | + | - | - | + |

### 2. In vivo expansion of the hybridomas for large scale production of Mab

Each hybridoma was expanded *in vitro* in RPMI + FCS medium using conventional methods, until a sufficient number of cells was obtained for i.p. injection of 3.10⁶ cells/mouse in 4 mice syngenic with those from which the myeloma and splenocytes were derived. The mice had been previously treated with two i.p. injections of 0.5 mL/mouse of pristane (2,6,10,14-tetramethyl-pentadecane, Aldrich Chem.) About 10 days after injection, collection of ascitic fluid was begun.

### 3. Purification of the monoclonal antibodies

Each ascitic fluid was purified by chromatography on Protein A Sepharose CL4B (Pharmacia, 17-0963-03); the average yield in IgG, taken as the ratio between total mg of IgG found and the total mL of ascitic fluid passed through the column, was 3 mg/mL.

### 4. Specificity of the purified Ig and titration curve on human IgG

In accordance with the ELISA method reported above (2.4.), the microtiter plates were coated with human IgG-IgA-IgM. For each class of immunoglobulins, a solid phase was prepared which represented a dilution curve starting from 20 µg/mL to 0.5 ng/mL by doubling dilutions. The results are reported in the table below. Another clone called 18-13, which is also specific for human IgG, was included in the test for comparison.

| Conc. µg/ml | IgG | | IgA | | IgM | |
|---|---|---|---|---|---|---|
| | 1* | 2 | 3* | 4 | 5* | 6 |
| | Optical density | | | | | |
| 20 | 2833 | 2578 | 127 | 197 | 270 | 348 |
| 2.5 | 2273 | 2416 | 78 | 106 | 198 | 265 |
| 0.6 | 2194 | 2375 | 68 | 73 | 188 | 247 |
| 0.3 | 2141 | 2282 | 57 | 72 | 169 | 228 |
| 0.15 | 2109 | 2074 | 57 | 72 | 176 | 271 |
| 0.03 | 1728 | 1979 | 65 | 78 | 135 | 219 |
| 0.007 | 1417 | 1809 | 64 | 71 | 156 | 188 |
| 0.003 | 1337 | 1590 | 69 | 61 | 148 | 160 |
| 0.001 | 970 | 1279 | 63 | 65 | 145 | 130 |
| 0.0005 | 542 | 981 | 66 | 68 | 96 | 137 |

| | | | | | | |
|---|---|---|---|---|---|---|
| * - clone IgG 18-13 used for comparison | | | | | | |

Columns 1 and 2 show the optical densities obtained when clones IgG 18-13 and IgG 38-77 respectively are reacted with human IgG bound to the solid phase at various concentrations. Columns 3 and 4 show the results found with the same clones when reacted with IgA, while columns 5 and 6 show the results of the reaction with IgM. The data prove that clone IgG 38-77 specifically recognizes human IgG.

### Example 2

### Detection of human IgG anti-Rubella virus with a direct one-step "sandwich" method

The antigen composed of a preparation of purified, inactivated Rubella virus, was coated on the wells of a microtiter plate (Labsystem). The wells were washed with a solution of buffered saline (PBS), then dried at 37°C for 90 minutes in a vacuum. This solid phase was incubated together with the test sample (20 µl) and the tracer (200 µl), composed of monoclonal IgG 38-77 labelled with peroxidase (HRP), for at least 30 minutes at 37°C. It was washed with a saline buffer solution containing a non ionic tensioactive agent (Brij 0.05%), after which the substrate (tetramethyl-benzidine and hydrogen peroxide solution in citrate buffer pH 3,8) was added. After 10 minutes incubation at room temperature, the reaction was stopped with H₂SO₄ 0.3 M. The optical density (OD) was read at 450 nm. The OD is proportional to the amount of anti-rubella IgG present in the sample. The results are reported in the following table.

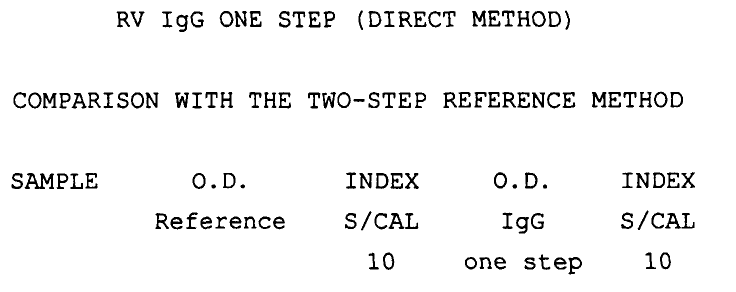

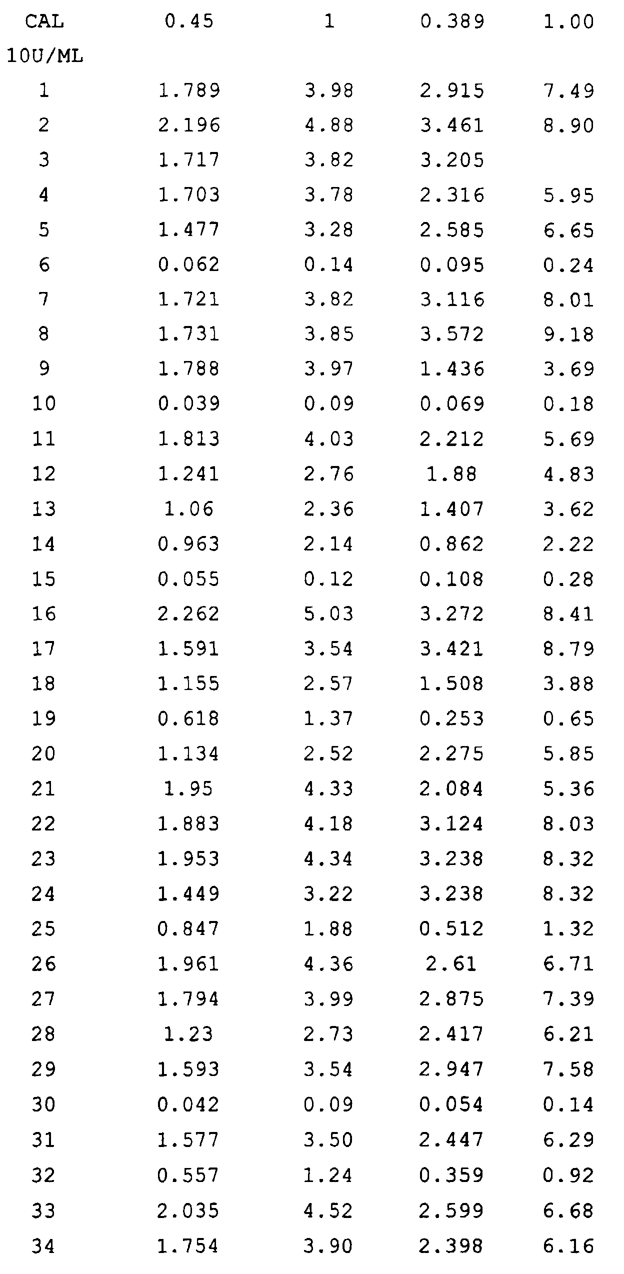

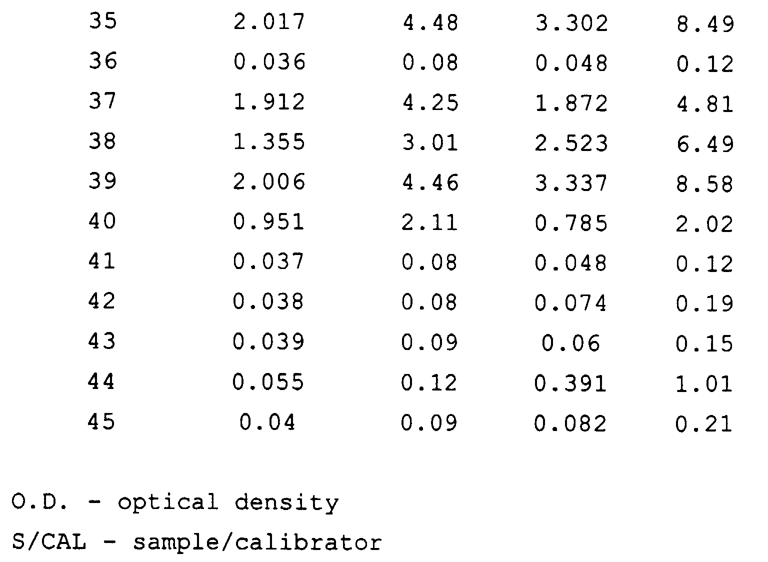

The table reports the experimental data compared with a method requiring classically two incubation steps. The kit used for the comparison is Rubella IgG (Diesse, cat. no. 91030, lot 102, expiry 01/2000). The calibrator at 10 IU/ml in accordance with the "3rd International Standard" is considered as test reference. The indexes are calculated as ratio between the OD of the sample and that of the reference. The results show that the two methods are very similar, with the advantages offered by the present invention.

Using the same solid phase described above (Rubella virus antigen), three negative and three positive samples were tested with the one-step technique, which requires that the clone be incubated with the sample for 15-60 minutes in a microplate well to which the antigen is bound in the preferential ratio 1 :10. The results are reported in the table below.

### Comparison between the anti-human IgG 38-77 clone and the 18-13 clone in the assay of human anti-Rubella IgG

### One-step technique

| Sample | 18-13 Clone labelled with HRP | IgG 38-77 Clone labelled with HRP |
|---|---|---|
| | OD | OD |
| 87 N | 0.166 | 0.111 |
| 7 N | 0.233 | 0.066 |
| 42 N | 0.268 | 0.043 |
| 103 P | 0.914 | 2.535 |
| 102 P | 0.829 | 2.817 |
| 101 p | 0.887 | 3.336 |
| OD = optical density | | |

The difference in optical density found when using the IgG 38-77 clone compared to another anti-human IgG clone (18-13) is evident. The comparison demonstrates that the IgG 38-77 clone binds the immune complexes between antigen and human IgG in a strongly preferential manner.

### Example 3

### Assay of anti-Rubella IgG with the capture technique in a single incubation

IgG 38-77 monoclonal antibodies diluted in bicarbonate buffer at pH 9.6 were coated on the wells of a microtiter plate (Labsystem), by incubation at room temperature for 18-26 hours. After washing with water and drying as reported in example 2, the solid phase is ready for the exclusive capture of the anti-rubella IgG that have reacted with the antigen. To perform the test, 50 µl of human serum diluted 1:51 with 50 µl of the immune complex that has been formed between the antigen (purified and inactivated Rubella virus) and the HRP conjugate of the anti-rubella 80-B clone (produced by Diesse and sold by catalogue number 93730).

After 30 minutes incubation the well is washed as reported in the previous example and then the substrate is added to detect the enzymatic activity bound to the well, which is proportional to the amount of human anti-Rubella IgG present in the test sample.

| RV IgG ONE STEP (CAPTURE OF THE IMMUNE COMPLEX) COMPARISON WITH THE REFERENCE METHOD | | | | |
|---|---|---|---|---|
| SAMPLE | Reference O.D. | INDEX S/CAL 10 | O.D. IgG one step | INDEX S/CAL 10 |
| CAL 10U/ml | 0.45 | 1.00 | 0.39 | 1.00 |
| 1 | 1.441 | 3.20 | 1.782 | 4.57 |
| 2 | 2.199 | 4.89 | 3.246 | 8.32 |
| 3 | 1.674 | 3.72 | 1.697 | 4.35 |
| 4 | 1.521 | 3.38 | 1.314 | 3.37 |
| 5 | 1.443 | 3.21 | 1.653 | 4.24 |
| 6 | 1.321 | 2.94 | 1.449 | 3.72 |
| 7 | 0.068 | 0.15 | 0.18 | 0.46 |
| 8 | 1.369 | 3.04 | 1.383 | 3.55 |
| 9 | 0.769 | 1.71 | 0.408 | 1.05 |
| 10 | 1.445 | 3.21 | 1.593 | 4.08 |
| 11 | 1.208 | 2.68 | 1.112 | 2.85 |
| 12 | 1.117 | 2.48 | 1.025 | 2.63 |
| 13 | 1.331 | 2.96 | 1.226 | 3.14 |
| 14 | 1.68 | 3.73 | 1.894 | 4.86 |
| 15 | 1.409 | 3.13 | 2.87 | 7.36 |
| 16 | 0.049 | 0.11 | 0.194 | 0.50 |
| 17 | 1.077 | 2.39 | 0.487 | 1.25 |
| 18 | 0.751 | 1.67 | 0.33 | 0.85 |
| 19 | 0.057 | 0.13 | 0.259 | 0.66 |
| 20 | 2.034 | 4.52 | 2.693 | 6.91 |
| 21 | 0.197 | 0.44 | 0.327 | 0.84 |
| 22 | 1.362 | 3.03 | 1.624 | 4.16 |
| 23 | 0.843 | 1.87 | 0.511 | 1.31 |
| 24 | 0.458 | 1.02 | 0.267 | 0.68 |
| 25 | 1.42 | 3.16 | 1.285 | 3.29 |
| 26 | 1.861 | 4.14 | 1.935 | 4.96 |
| 27 | 1.681 | 3.74 | 1.961 | 5.03 |
| 28 | 0.043 | 0.10 | 0.187 | 0.48 |
| 29 | 0.041 | 0.09 | 0.228 | 0.58 |
| 30 | 0.051 | 0.11 | 0.211 | 0.54 |
| O.D. = Optical density S/CAL - sample/calibrator | | | | |

The table reports the results found with 30 samples. The reference is represented by the calibrator at 10 IU/mL. The index is calculated as in the previous example. The reference kit was the same as that used in the previous example. Also in this case there is marked similarity between the results, which are however obtained in a simpler and more rapid manner by means of the invented method.

## Claims

1. Enzyme immunoassay for the qualitative and quantitative determination of class IgG human antibodies in a test sample, including the use of a monoclonal antibody which reacts selectively with the IgG, in which said monoclonal antibody is directed towards a conformational epitope of the Fc fragment of human IgG which is exposed by binding to an antigen specific for the IgG and it binds selectively to the IgG which have reacted with said antigen.

2. A method according to claim 1, which includes coating of an antigen specific for the IgG on a solid phase, contact between the resulting solid phase and the test sample, in a mixture with the monoclonal antibody to which a tracer is bound either directly or indirectly, incubation of the solid phase including the mixture of the sample and the monoclonal/tracer, and washing of the resulting final mixture.

3. A method according to claim 1, including coating of the monoclonal antibody on a solid phase; the mixing of the antigen, to which a tracer is bound either directly or indirectly, with the sample; incubation of the solid phase including the sample/antigen-tracer mixture; and washing of the final resulting mixture.

4. A method according to claims 2 or 3, which includes a single incubation step and a single washing step.

5. A method according to any of the claims 2 to 4, in which the tracer is an enzyme.

6. A method according to claim 5, in which the enzyme is peroxidase HRP.

7. A method according to any of the previous claims, in which the monoclonal is produced by means of the hybridoma IgG 38-77.

8. A monoclonal antibody which binds selectively to the human IgG which have reacted with an antigen specific for the IgG and directed towards a conformatiorial epitope of the fragment Fc of the IgG which is exposed after binding with the antigen.

9. An antibody according to claim 8, produced by means of the hybridoma IgG 38-77.

10. A reagent for the qualitative and quantitative enzyme immunoassay of class IgG human antibodies, which includes the monoclonal antibody reported in claims 8 or 9.

11. A kit for the qualitative and qunatitative enzyme immunoassay of human class IgG antibodies which includes the reagent reported in claim 10.

12. A kit according to claim 11, in which a tracer is bound, either directly or indirectly, to the reagent.

13. A kit according to claim 12, in which the tracer is an enzyme.

14. A kit according to claim 13, in which the enzyme is HRP.
